(19) Europäisches Patentamt
European Patent Office
Office européen des brevets

(11) EP 0 479 093 B1

(12) EUROPÄISCHE PATENTSCHRIFT

(45) Veröffentlichungstag und Bekanntmachung des Hinweises auf die Patenterteilung:
**31.01.1996 Patentblatt 1996/05**

(51) Int Cl.$^6$: **C07H 11/00**, A61K 31/70, A61K 31/715

(21) Anmeldenummer: **91116184.2**

(22) Anmeldetag: **24.09.1991**

# (54) Oligosaccharidderivate und deren Verwendung in Heilmitteln

Oligosaccharide derivatives and their use as medicaments

Dérivés oligosaccharides et leurs utilisations comme médicaments

(84) Benannte Vertragsstaaten:
**AT BE CH DE DK FR GB IT LI LU NL SE**

(30) Priorität: **03.10.1990 CH 3177/90**
**30.07.1991 CH 2267/91**

(43) Veröffentlichungstag der Anmeldung:
**08.04.1992 Patentblatt 1992/15**

(73) Patentinhaber: **F. HOFFMANN-LA ROCHE AG**
**CH-4002 Basel (CH)**

(72) Erfinder:
- **Hosang, Markus Dr.**
  **CH-4123 Allschwil (CH)**
- **Iberg, Niggi**
  **CH-4059 Basel (CH)**
- **Trumtel, Michel**
  **F-45500 Gien (FR)**
- **Tschopp, Thomas B.**
  **CH-4107 Ettingen (CH)**
- **Wessel, Hans Peter**
  **W-7843 Heitersheim 2 (DE)**

(74) Vertreter: **Grossner, Lutz, Dr. et al**
**CH-4002 Basel (CH)**

(56) Entgegenhaltungen:
**EP-A- 0 230 023** **EP-A- 0 301 618**
**GB-A- 1 381 426** **US-A- 4 359 458**

- **BIOCHEMISTRY, Band 15, Nr. 13, 1976, EASTON, PA,**
- **US; Seiten 2728-2735; M.B.GOREN ET AL.: 'Sulfatides of Mycobacterium Tuberculosis: The Structure of the Principal Sulfatide (SL-I)'**
- **CHEMICAL ABSTRACTS, Band 101, Nr. 13, 24. September 1984, Columbus, Ohio, US; Zusammenfassung Nr. 111299C: A.LIAV ET AL.: 'Sulfatides of Monobacterium tuberculosis. Synthesis of the core alpha, alpha-Trehalose 2- sulfate'; Seite 692, Spalte 1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 479 093 B1

Printed by Jouve, 75001 PARIS (FR)

## Beschreibung

Die vorliegende Erfindung betrifft neue sulfatierte Oligosaccharide der Formel

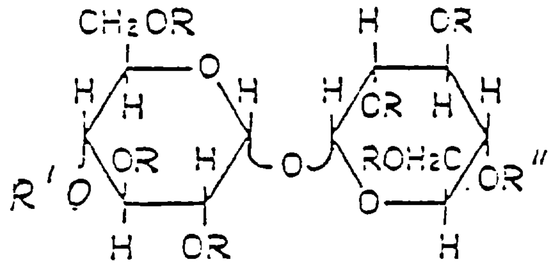

I

worin R Wasserstoff oder ein Rest $-SO_3M$; M ein Kation; R' ein equatorial oder quasiequatorial verknüpfter sulfatierter Mono-, Di- oder Trisaccharidrest; oder ein axial verknüpfter sulfatierter Trisaccharidrest; und R" Wasserstoff oder ein equatorial oder quasiequatorial verknüpfter sulfatierter Mono- oder Disaccharidrest ist; wobei das Molekül höchstens 6 Monosaccharideinheiten enthält und im Mittel mindestens eine $-SO_3M$-Gruppe pro Monosaccharideinheit anwesend ist.

Die Erfindung betrifft weiterhin ein Verfahren zur Herstellung der Verbindungen der Formel I, ihre Verwendung als Heilmittel bzw. als Wirkstoffe zur Herstellung von pharmazeutischen Präparaten und pharmazeutische Präparate auf der Basis von Verbindungen der Formel I.

Es ist bekannt, Schwefelsäureester von Oligosacchariden zur Behandlung von Arteriosclerose einzusetzen. Es kann in diesem Zusammenhang auf die britische Patentanmeldung GB 1381426 verwiesen werden. Die dort eingesetzten Schwefelsäureester bestehen aus Glucose-Einheiten, die α-1,4 oder α-1,6 verknüpft sind, wogegen die erfindungsgemässen sulfatierten Oligosaccharide zwei 1,1 verknüpfte Glucose-Einheiten enthalten.

Als Kation M kommen alle physiologisch verträglichen Kationen in Betracht, z.B. Alkalimetallkationen wie $Na^+$ und $K^+$; Ammoniumionen und substituierte Ammoniumionen, die sich von tertiären Aminen wie Triäthylamin, oder Pyridin oder Imidazol ableiten; oder quaternäre Ammoniumionen wie Dodecyltrimethylammonium, Aethylpyridinium und Benzethonium; sowie Erdalkalimetallkationen wie $Ca^{++}$. Bevorzugt sind Verbindungen, in denen M $Na^+$ ist.

Als Sulfatierungsgrad wird die Anzahl von $-SO_3M$-Resten pro Monosaccharideinheit bezeichnet, die im Molekül im Mittel vorhanden sind. Der Sulfatierungsgrad 1 liegt somit z.B. dann vor, wenn ein Hexasaccharid der Formel I 6 $-SO_3M$-Reste im Molekül enthält. Vorzugsweise beträgt der Sulfatierungsgrad in den Verbindungen der Formel I 2-3.

Equatorial verknüpfte sulfatierte Mono-, Di- oder Trisaccharidreste R' sind vorzugsweise β-glykosidisch verknüpfte Reste; jedoch kann eine equatoriale Verknüpfung, z.B. bei Arabinoseglykosiden, mit einer α-glykosidischen Bindung des Restes R' an den Trehaloserest einhergehen. Die Bezeichnung "quasiequatorial" bezieht sich auf die Konformation von Furanosiden.

Die Verbindungen der Formel I können erfindungsgemäss dadurch hergestellt werden, dass man ein entsprechendes Tri-, Tetra-, Penta- oder Hexasaccharid mit einem Sulfatierungsmittel behandelt und das Reaktionsprodukt in ein Salz überführt bzw. als solches isoliert. Beispiele von Monosaccharidresten sind Glucopyranosyl, Mannopyranosyl, Arabinopyranosyl, Galactofuranosyl, Arabinofuranosyl, Ribofuranosyl und Rhamnofuranosyl. Beispiele von Disaccharidresten sind Maltosyl, Cellobiosyl, Lactosyl, Melibiosyl, Gentiobiosyl und Galactopyranosidoarabinopyranosyl. Ein Trisaccharidrest ist beispielsweise Maltotriosyl. Die vorstehend genannten Reste sind als Substituenten R' oder R" im Rahmen der Definition der Formel I sulfatiert. Beispiele für Verbindungen der Formel I sind die Verbindungen der Formeln Ia und Ib, wobei R die oben angegebene Bedeutung hat und im Mittel mindestens ein Rest R pro Monosaccharideinheit $-SO_3M$ ist. In der Verbindung Ia ist R' β-D-Maltotriosyl und R" Wasserstoff, in der Verbindung Ib sind R' und R" β-D-Maltosyl.

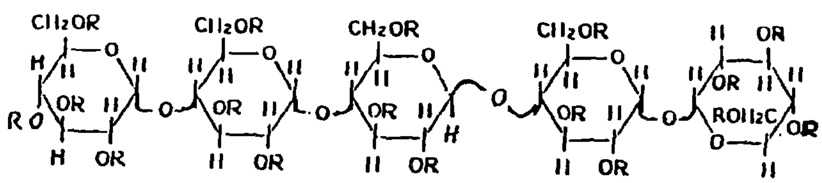

Ia

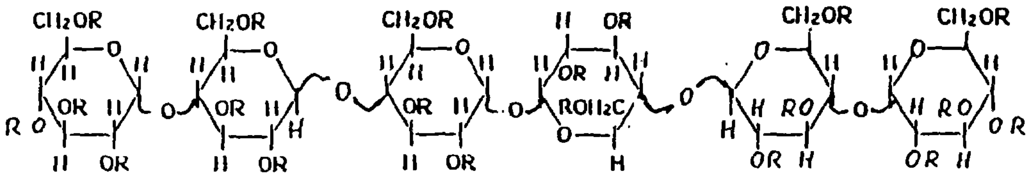

Ib

Die Sulfatierung gemäss der vorliegenden Erfindung kann mittels Methoden, die für die Sulfatierung von Hydroxygruppen an sich bekannt sind, vorgenommen werden.

Beispiele von Sulfatierungsmitteln, die für die Herstellung der Verbindungen der Formel I angewandt werden können, sind $SO_3$"Komplexe wie $SO_3$·Pyridin, $SO_3$·Trimethylamin, $SO_3$·Dioxan und $SO_3$·Dimethylformamid. Weitere Beispiele von Sulfatierungsmitteln sind Chlorsulfonsäure, Gemische von Chlorsulfonsäure und Schwefelsäure; und Piperidin-N-Sulfat.

Die Umsetzung erfolgt zweckmässig in einem geeigneten Lösungsmittel, insbesondere einem polaren Lösungsmittel, z.B. Dimethylformamid, Dimethylsulfoxid oder Hexamethylphosphortriamid. Die Reaktion kann bei Raumtemperatur oder erhöhter Temperatur, z.B. bei 20-70°C durchgeführt werden, wobei durch Variation der Reaktionsdauer und -temperatur der Sulfatierungsgrad beeinflusst werden kann. Der jeweils erreichte Sulfatierungsgrad kann durch HPLC abgeschätzt werden. Die Aufarbeitung des Reaktionsgemisches bzw. die Isolierung des Reaktionsprodukts der Formel I kann nach an sich bekannten Methoden aus dem Reaktionsgemisch erfolgen, z.B. durch Gelfiltration oder Ultrafiltration.

Die als Ausgangsmaterialien eingesetzten freien Saccharide sind bekannt oder nach im Prinzip bekannten Methoden zugänglich. Für die Herstellung kommen enzymatische oder synthetische chemische Verfahren in Betracht. Die Oligosaccharide sind prinzipiell mit sequentiellen Synthesen oder Blocksynthesen aufbaubar. Hierbei werden glykosidische Bindungen durch Reaktion eines Glykosylakzeptors mit einem Glykosyldonor in Gegenwart eines geeigneten Katalysators hergestellt. Als Glykosyldonoren sind derivatisierte Glykosylverbindungen geeignet, die am anomeren Zentrum aktiviert sind, wie z.B. Chloride, Bromide, Fluoride, Acetate, Trichloracetimidate, Alkylthioderivate, usw.

Als Glykosylakzeptoren sind solche Saccharidderivate geeignet, bei denen die zu glykosidierenden OH-Gruppen frei und die übrigen vollständig oder teilweise geschützt sind. Bei nur teilweisem Schutz der übrigen OH-Gruppen kann die Glykosidierung selektiv erfolgen oder durch eine unterschiedliche Umgebung der Hydroxylgruppen in eine bestimmte Richtung gelenkt werden.

Die Verbindungen der Formel I hemmen die Migration und Proliferation von Zellen der vaskulären glatten Muskulatur und verhüten proliferative arteriosklerotische Läsionen. Ihre blutgerinnungshemmende Aktivität ist geringer als die von Heparin. Insbesondere haben die Verbindungen in vitro keine anticoagulante Aktivität, d.h. sie haben keinen oder nur sehr geringen Einfluss auf die Gerinnungsfaktoren Thrombin (F.IIa) und F.Xa. Die Verbindungen der Formel I können daher zur Prophylaxe von arteriosklerotischen Erkrankungen, insbesondere nach Bypass-Operationen oder Angioplastie, sowie zur Behandlung von Patienten mit progressiver Arteriosklerose verwendet werden.

Die blutgerinnungshemmende Wirkung wurde wie folgt ermittelt:

<u>aPTT (activated partial thromboplastin time)-Test</u> (vgl. Walenga et al., CRC Critical Reviews in Laboratory Sciences 22 (4) 361-389 (1986)): 100 ml zitriertes menschliches Plasma, das verschiedene Konzentrationen der Versuchsverbindung enthält, wird mit 100 ul Activated Thrombofax® (Ortho Diagnostics, Raritan, N.J., U.S.A.) 8 Minuten bei 37°C inkubiert. Dann werden 100 µl vorgewärmte 25 mM Calciumchloridlösung zugesetzt und die Gerinnungszeit wird im Fibrometer Coagulation Timer (Becton, Dickinson Basel) gemessen.

<u>anti-Xa Clotting Assay:</u> 25 ml zitriertes Plasma mit verschiedenen Konzentrationen der Versuchsverbindung werden mit 75 µl 1:100 verdünntem Factor Xa (Diagnostic Reagents, Thame, Oxon, Grossbritannien) in 0,63% Zitratpuffer (pH 7,3), der 41 mM Imidazol, 82 mM NaCl und 0,1% Albumin enthält, gemischt. Nach 2 Minuten Erwärmen auf 37°C werden 200 µl eines 1:1-Gemisches von Factor X Mangel-Plasma (Diagnostic Reagents) und Platelet Substitute (Diagnostic Reagents) zugesetzt und das Gemisch 20 Sekunden bei 37°C inkubiert. Nach Zusatz von 100 µl vorgewärmter 50 mM Calciumchloridlösung wird die Gerinnungszeit im Fibrometer gemessen.

Die Aktivität der Versuchsverbindung wird als $IC_{50}$ angegeben, die jene Konzentration [µg/ml] angibt, welche zu einer Gerinnungszeit führt, die das Doppelte des Kontrollwerts beträgt.

<u>Hemmung von Thrombin oder Faktor Xa im Chromogenic Substrate Assay</u> (Teien et al., Thrombosis Research 10, 399-410 (1977)): Die Bestimmung erfolgte im Cobas-Bio Centrifugal Automatic Spectrophotometer. Die verwendete Pufferlösung bestand aus 50 mM Tris-Puffer, 180 mM NaCl, 7,5 mM EDTA-$Na_2$, 1% PEG 6000 und 0,02% Tween-80, pH 8,4. Die Testlösung bestand aus 50 µl Puffer, 30 µl Antithrombin III (1 U/ml, Kabi-Diagnostica) und 20 µl Plasma, das verschiedene Konzentrationen der Versuchsverbindungen enthielt. Im Analysenautomat wurden 30 µl Probenlösung und 20 ml Wasser mit 180 µl Thrombin (1 U/ml, Thrombin Reagent Roche Basel) in die Testküvette gegeben.

Nach 240 Sekunden Inkubation bei 37°C wurden 60 µl S-2238 (H-D-Phe-Pip-Arg-NH.pNA, Kabi Diagnostica, Möndal, Schweden, 0,75 mM in Wasser) und 20 ml Wasser zugesetzt. Die Freisetzung von pNA (p-Nitroanilin) wurde während 60 Sekunden bei 405 nm in 10-Sekunden-Intervallen im Vergleich zu Wasser als Blindwert verfolgt. Die Hemmwirkung wird als $IC_{50}$ angegeben, was die Konzentration [µg/ml] angibt, bei der die amidolytische Aktivität von Thrombin im Vergleich zum Plasma-Kontrollwert um 50% vermindert wird.

In gleicher Weise wurde die Hemmung von Faktor Xa gemessen, wobei eine Lösung von Faktor Xa (2,8 nkat/ml und 2 mM S-2222 (Bz-CO-Ile-Glu-Arg-NH.pNA, Kabi Diagnostica) in Wasser anstelle von Thrombin bzw. S-2238 verwendet wurde.

Die anti-proliferative Aktivität der Substanzen wurde in Zellkulturen wie folgt ermittelt: Glatte Muskelzellen der Ratte (in DMEM mit 10% FCS bei 37°C und 5% $CO_2$ kultiviert) wurden auf Zellkulturplatten mit einer Dichte von $8x10^3$ Zellen/Vertiefung ausgebracht. Nach 4 Stunden wurde die Zahl der adherierten Zellen bestimmt und die zu testenden Substanzen (100 µg/ml) zugegeben. Als Vergleich dienten Zellen, denen nichts zugegeben wurde, und als positive Kontrolle diente Heparin (100 µg/ml). Die Zellen wurden für 7 Tage inkubiert und dann die Zellzahl bestimmt. Die anti-proliferative Aktivität der einzelnen Substanzen wurde als % Inhibition im Vergleich zum nichtinhibierten Wachstum errechnet:

$$\% \text{ Inhibition} = \frac{\text{Zellzahl}_{(-)} - \text{Zellzahl}_{(inhib)}}{\text{Zellzahl}_{(-)} - \text{Zellzahl}_{(d=0)}} * 100$$

wobei

| | |
|---|---|
| $\text{Zellzahl}_{(d=0)}$ = | Zellzahl nach 4 h |
| $\text{Zellzahl}_{(-)}$ = | Zellzahl der Zellen denen nichts zugegeben wurde, nach 7 Tagen |
| $\text{Zellzahl}_{(inhib)}$ = | Zellzahl der Zellen mit 100 mg/ml der zu testenden Substanz |

Die Resultate, die in den oben beschriebenen Versuchsanordnungen mit Verbindungen der Formel I erhalten wurden, sind in Tabelle 1 aufgeführt. Als Referenzverbindung diente Heparin.

Tabelle I

| Verbindungvon Beispiel | anti-proliferative Aktivität % Hemmung | Anti-Koagulationswirkung $IC_{50}$ [µg/ml] Gerinnungshemmung amidolytische Aktivität | | | |
|---|---|---|---|---|---|
| | | aPTT | Xa | Thrombin | F.Xa |
| 1G. | 57 | 11 | 31 | >1000 | 960 |
| 2C. | 38 | 40 | 115 | >1000 | >1000 |
| 3C. | 59 | 12 | 33,5 | >1000 | >1000 |
| 4C. | 64 | 11 | 32 | >1000 | >1000 |
| 5C. | 39 | 33 | 69 | >1000 | >1000 |
| 6D. | 67 | 14,5 | 28,5 | >1000 | 900 |
| 7C. | 49 | 13,5 | 34 | >1000 | >1000 |
| Heparin | 47 | 1,2 | 0,6 | 1,9 | 2,7 |

Die Versuchsresultate zeigen, dass die erfindungsgemässen Verbindungen eine anti-proliferative Wirkung aufweisen, die im Gegensatz zu dem ebenfalls anti-proliferativ wirksamen Heparin nicht oder in viel geringerem Mass von einer anti-Koagulationswirkung begleitet ist.

Die Arzneimittel auf der Basis der erfindungsgemässen Verbindungen können enteral, z.B. oral in Form von Tabletten, Lacktabletten, Dragees, Hart-und Weichgelatinekapseln, Lösungen, Emulsionen oder Suspensionen, oder rektal, z.B. in Form von Suppositorien, verabreicht werden. Die Verabreichung erfolgt jedoch vorzugsweise parenteral, z.B. in Form von Injektionslösungen.

Zur Herstellung von Tabletten, Lacktabletten, Dragèes und Hartgelatinekapseln kann der Wirkstoff mit pharmazeutisch inerten, anorganischen oder organischen Excipientien vermischt werden. Als solche Excipientien kann man für Tabletten, Dragèes und Hartgelatinekapseln z.B. Lactose, Maisstärke oder Derivate davon, Talk, Stearinsäure oder deren Salze, verwenden. Für Weichgelatinekapseln eignen sich als Excipientien z.B. vegetabile Oele, Wachse, Fette, halbfeste und flüssige Polyole; je nach Beschaffenheit des Wirkstoffes sind jedoch bei Weichgelatinekapseln überhaupt keine Excipientien erforderlich. Zur Herstellung von Lösungen und Sirupen eignen sich als Excipientien z.B. Wasser, Polyole, Saccharose, Invertzucker und Glukose, für Injektionslösungen eignen sich z.B. Wasser, Alkohole, Polyole, Glycerin und vegetabile Oele, und für Suppositorien eignen sich z.B. natürliche oder gehärtete Oele, Wachse, Fette und

halbflüssige oder flüssige Polyole.

Die pharmazeutischen Präparate können daneben noch Konservierungsmittel, Lösungsvermittler, Stabilisierungsmittel, Netzmittel, Emulgiermittel, Süssmittel, Färbemittel, Aromatisierungsmittel, Salze zur Veränderung des osmotischen Druckes, Puffer, Ueberzugsmittel oder Antioxidantien enthalten. Bei enteraler Verabreichung kann die Resorption des Wirkstoffs mit Hilfe von Liposomen erhöht werden.

Die Dosierung des Wirkstoffes kann innerhalb weiter Grenzen variieren und ist natürlich in jedem einzelnen Fall den individuellen Gegebenheiten anzupassen. Im allgemeinen dürfte bei parenteraler Verabreichung eine Dosis von etwa 0,1 bis 100 mg/kg, vorzugsweise von etwa 1,5 bis 15 mg/kg, pro Tag für den Erwachsenen angemessen sein, wobei aber die soeben angegebene obere Grenze auch überschritten werden kann, wenn sich dies als angezeigt erweisen sollte.

Beispiel 1

A. Eine Lösung von 28,5 g 2,2',3,3'-Tetra-O-benzyl-4,6-O-benzyliden-α,α-D-trehalose (Carbohydr. Res. 63, 51 (1978)) und 27 ml Bistributylzinnoxid in 2,2 l Toluol wurde 4,5 Stunden am Wasserabscheider unter Rückfluss erhitzt und auf ein Volumen von 800 ml reduziert. Anschliessend wurden 3,84 g Tetrabutylammoniumbromid und 42,8 ml Benzylbromid zugegeben und bei 100°C weitergerührt. Nach 16 Stunden wurde die Lösung abgekühlt und extrahierend in einem Natriumhydrogencarbonat/Methylenchlorid-System aufgearbeitet. Die vereinigten organischen Phasen wurden getrocknet ($MgSO_4$) und an Kieselgel mit Aceton/Hexan 1:2 (enthaltend 1‰ Triäthylamin) als Laufmittel chromatographiert. Produktfraktionen wuden kristallisiert und ergaben 27,5 g (86%) 2,2',3,3',6'-Penta-O-benzyl-4,6-O-benzyliden-α,α-D-trehalose, Smp. 122°C.

B. Eine Lösung von 32,7 g Deca-O-acetyl-α-D-maltotriosylbromid (K. Tabeo, K. Mine und T. Kuge, Carbohydr. Res. 48, 197 (1976)), in 85 ml Allylalkohol wurde in Gegenwart von 8,5 g Quecksilber-II-cyanid 90 Minuten bei 50-60°C gerührt, aufkonzentriert und in ätherischer Lösung nacheinander mit 1 molarer Kaliumjodidlösung, Natriumbicarbonatlösung und Wasser extrahiert. Chromatographie des Rohproduktes an Kieselgel mit Essigester/Hexan 1:1 als Elutionsmittel ergab 25,3 g (79,6%) reines Allyl-deca-O-acetyl-β-D-maltotriosid, $[\alpha]^{20}_{D}$ = +75,0° (c = 0,5, Dioxan).

C. Eine Lösung von 24,8 g Allyl-deca-O-acetyl-β-D-maltotriosid in 150 ml Methanol wurde in Gegenwart von katalytischen Mengen wasserfreiem Natriumcarbonat 18 Stunden bei Raumtemperatur gerührt, dann filtriert und mit saurem Ionenaustauscher neutralisiert. Nach Abziehen des Lösungsmittels wurde in 350 ml Dimethylformamid aufgenommen und bei Raumtemperatur mit 8,0 g Natriumhydrid (80% in Weissöl) versetzt. Nach 90 Minuten Rühren wurde auf 1°C abgekühlt, mit 32 ml Benzylbromid versetzt und 30 Minuten unter Eiskühlung und 1 Stunde bei Raumtemperatur gerührt. Danach wurde 100 ml Methanol zugetropft und 1 Stunde ausgerührt. Das Reaktionsgemisch wurde eingeengt, in Essigester aufgenommen und mit wässriger Natriumbicarbonatlösung und mit Wasser extrahiert. Die organischen Phasen wurden getrocknet und eingeengt (38,5 g). 5 g benzylierten Rohproduktes wurde in 40 ml Essigsäure/Wasser 9:1 suspendiert und in Gegenwart von 2,46 g Palladiumchlorid und 2,46 g Natriumacetat 3 Stunden im Ultraschallbad behandelt. Danach wurde abgenutscht, gewaschen, eingedampft, der Rückstand in Essigester aufgenommen und die Lösung nacheinander mit wässriger Natriumbicarbonatlösung und Wasser gewaschen. Die Essigesterphasen wurden getrocknet ($Na_2SO_4$), eingeengt und an Kieselgel mit Toluol/Essigester 7:1 als Elutionsmittel chromatographiert. Es wurden 3,99 g (90%) sirupöse Deca-O-benzyl-D-maltotriose erhalten.

D. Zu einer Lösung von 3,69 g Deca-O-benzyl-D-maltotriose in 25 ml absolutem Dichlormethan und 0,1 ml absolutem Dimethylformamid wurde während 45 Minuten bei 3°C eine Lösung von 0,25 ml Oxalylchlorid in 7 ml absolutem Dichlormethan zugetropft. Nach 6 Stunden Rühren bei Raumtemperatur wurde eingedampft. Das getrocknete rohe Deca-O-benzyl-α-D-maltotriosylchlorid wurde in 10 ml absolutem Acetonitril gelöst und in Gegenwart von 0,97 g getrocknetem Silberfluorid 3 Stunden bei Raumtemperatur gerührt. Das Reaktionsgemisch wurde filtriert und der Niederschlag mit Aether gewaschen. Die organischen Lösungen wurden mit wässriger gesättigter Natriumchloridlösung versetzt und 15 Minuten stark gerührt. Nach Abnutschen des ausgefallenen Niederschlages wurde eingeengt, mit Aether verdünnt und nacheinander mit Natriumchloridlösung und Wasser gewaschen. Die ätherischen Lösungen wurden eingedampft. Chromatographie an Kieselgel mit Essigester/Hexan 1:6 als Elutionsmittel ergab 2,19 g (66%) reines Deca-O-benzyl-β-D-maltotriosylfluorid, $[\alpha]^{20}_{D}$ = 58,9° (c = 0,9, $CHCl_3$).

E. Zu einer Lösung von 1,69 g gut getrocknetem Deca-O-benzyl-β-D-maltotriosylfluorid und 538 mg 2,2',3,3',6'-Penta-O-benzyl-4,6-O-benzyliden-a,α-D-trehalose in 20 ml trockenem Aether wurde in Gegenwart von

Molekularsieb (4Å) innerhalb 1 Stunde bei -20°C eine Lösung von 110 ml Trifluormethansulfonsäureanhydrid in 5 ml trockenem Aether zugetropft. Es wurde auf Raumtemperatur erwärmt und 48 Stunden gerührt. Nach Filtration über Filterhilfe, Waschen mit Aether und Einengen wurde an Kieselgel Toluol/Essigester 14:1 als Laufmittel chromatographiert. Es wurden 414 mg (30%) 2,2',3,3',6'-Penta-O-benzyl-4'-O-(deca-O-benzyl-α-D-maltotriosyl) -4,6-O-benzyliden-α,α-D-trehalose als sirupöses Produkt erhalten, $[\alpha]_D^{20}$ = +44° (c = 0,0846, EtOH).

F. Eine Lösung von 295 mg 2,2',3,3',6'-Penta-O-benzyl-4'-O-(deca-O-benzyl-α-D-maltotriosyl)-4,6-O-benzyliden-α,α-D-trehalose in 10 ml Aethanol und 5 ml Wasser wurde in Gegenwart von 300 mg 10% Palladium auf Kohle bei Raumtemperatur 2 Stunden hydriert. Die Reaktionslösung wurde über Filterhilfe filtriert, nachgewaschen und gefriergetrocknet. Das Rohprodukt wurde mit Wasser (an Sephadex® LH20) gelchromatographiert und das Produkt lyophilisiert. Man erhielt 65 mg 4-O-(α-D-Maltotriosyl)-α,α-D-trehalose, $[\alpha]_D^{20}$ = +179,5° (c = 0,2, $H_2O$).

G. Eine Lösung von 30 mg 4-O-(α-D-Maltotriosyl)-α,α-D-trehalose in 1 ml absolutem Dimethylformamid wurden in Gegenwart von 170 mg Schwefeltrioxid-Trimethylaminkomplex 20 Stunden bei 50°C gerührt, wobei ein zäher Sirup ausfiel. Das Lösungsmittel wurde abdekantiert, der Rückstand wurde mit Methanol gewaschen, in 1,5 ml 10%iger Natriumacetatlösung gelöst und eingeengt. Der Rückstand wurde mehrfach in Wasser aufgenommen und zur Entfernung von Trimethylamin eingedampft. Der Rückstand wurde zur Entfernung von Salzen gelchromatographiert (Sephadex® LH 20). Nach Gefriertrocknung wurden 67 mg sulfatierte 4-O-(α-D-Maltotriosyl)-α,α-D-trehalose erhalten, S= 18,66%,DS (durchschnittlicher Sulfatierungsgrad) etwa 2,4.

Beispiel 2

A. Zu einer Lösung von 3,0 g getrockneter 2,2',3,3',6'-Penta-O-benzyl-4,6-O-benzyliden-α,α-D-trehalose in 30 ml absolutem Dichlormethan und 2,5 ml absolutem Tetramethylharnstoff wurde in Gegenwart von 2,61 g getrocknetem Silbertrifluormethansulfonat bei -30°C eine Lösung von 4,2 g α-D-Acetobromglucose in absolutem Dichlormethan zugetropft. Nach 20 Stunden Rühren bei Raumtemperatur wurde bei -30°C eine weitere Zugabe von 3,44 g α-D-Acetobromglucose in 50 ml Dichlormethan, 2ml Tetramethylharnstoff und 1,75 g Silbertrifluormethansulfonat vorgenommen. Nach 18 Stunden Rühren bei Raumtemperatur wurde filtriert und mit Dichlormethan gewaschen. Die organischen Lösungen wurden mit Natriumbicarbonatlösung und Wasser gewaschen und über Magnesiumsulfat getrocknet. Chromatographie an Kieselgel mit Toluol/Aether 6:1 als Elutionsmittel ergab 64% reine 4'-O-(2,3,4,6-Tetra-O-acetyl-β-D-glucopyranosyl)-2,2',3,3',6'-penta-O-benzyl-4,6-O-benzyliden-α,α-D-trehalose als Sirup, $[\alpha]_D^{20}$ = +53,0° (c = 0,2, Dioxan).

B. Eine Lösung von 1,14 g 4'-O-(2,3,4,6-Tetra-O-acetyl-β-D-glucopyranosyl)-2,2',3,3',6'-penta-O-benzyl-4,6-O-benzyliden-α,α-D -trehalose in 60 ml absolutem Methanol und 20 ml absolutem Cyclohexan (20 ml) wurde mit 1,4 ml 2%iger Natriummethylatlösung versetzt. Nach 4 Stunden bei Raumtemperatur wurde mit saurem Ionenaustauscher neutralisiert, filtriert, eingeengt und an Kieselgel mit Essigester/Methanol/Wasser 95:1:1 als Elutionsmittel chromatographiert, wobei 840 mg Produkt erhalten wurden. Ein 370 mg-Aliquot wurde in 60 ml Aethanol/Wasser 5:1 in Gegenwart von 10% Palladium auf Kohle bei Raumtemperatur hydriert. Nach 90 Minuten wurde filtriert, mit Aethanol und Wasser gewaschen und eingeengt. Es wurde eine quantitative Ausbeute an 4-O-(β-D-Glucopyranosyl) -α,α-D-trehalose erhalten, $[\alpha]_D^{20}$=+121,9° (c = 0,2, $H_2O$).

C. Eine Lösung von 840 mg gut getrockneter 4-O-(β-D-Glucopyranosyl)-α,α-D-trehalose in 30 ml absolutem Dimethylformamid wurden in Gegenwart von 5,08 g Schwefeltrioxid-Trimethylaminkomplex 20 Stunden bei 50°C gerührt, wobei ein zäher Sirup ausfiel. Das Lösungsmittel wurde abdekantiert, der Rückstand mit Methanol gewaschen, in 30 ml 10% Natriumacetatlösung gelöst und eingeengt. Der Rückstand wurde mehrfach in Wasser aufgenommen und zur Entfernung von Trimethylamin eingedampft. Der Rückstand wurde zur Entfernung von Salzen gelchromatographisch (Sephadex® LH20) gereinigt. Nach Gefriertrocknung wurde 2,0 g sulfatierte 4-O-(β-D-Glucopyranosyl) -α,α-D-trehalose erhalten, S = 20,40%, DS etwa 3,0.

Beispiel 3

A. Zu einer Lösung von 1,0 g gut getrockneter 2,2',3,3',6'-Penta-O-benzyl-4,6-O-benzyliden-α,α-D-trehalose in 6 ml absolutem Dichlormethan und 0,22 ml Tetramethylharnstoff wurde in Gegenwart von 0,43 g Silbertrifluormethansulfonat bei 0°C eine Lösung von 1,19 g Hepta-O-acetyl-α-D-maltosylbromid (J. Chem. Soc. 1962, 2823) in 5 ml absolutem Dichlormethan zugetropft. Nach 8 Stunden bei Raumtemperatur wurde eine Zugabe von 109 mg Silbertriflat, 0,05 ml Tetramethylharnstoff und 298 mg Maltosylbromid vorgenommen. Nach 18 Stunden Rühren bei Raumtemperatur wurde filtriert und mit Dichlormethan gewaschen. Die vereinigten organischen Lösungen wurden mit Natriumbicarbonatlösung und Wasser gewaschen und über Magnesiumsulfat getrocknet. Chromatographie an Kieselgel mit Essigester/Hexan 1:1 als Elutionsmittel ergab 1,49 g (88%) reine 4'-O-(Hepta-O-acetyl-β-D-maltosyl) -2,2',3,3',6'-penta-O-benzyl-4,6-O-benzyliden-α,α-D-trehalose als Sirup, $[\alpha]_D^{20}$ = +84,0° (c = 0,2, Dioxan).

B. Eine Lösung von 1,43 g 4'-O-(Hepta-O-acetyl-β-D-maltosyl)-2,2',3,3',6'-penta-O-benzyl-4,6-O-benzyliden-α,α-D-trehalose in 15 ml absolutem Methanol und 4 ml absolutem Cyclohexan wurde mit 6 ml 2%iger Natriummethylatlösung versetzt. Nach 15 Minuten bei Raumtemperatur wurde mit saurem Ionenaustauscher neutralisiert, filtriert und eingeengt. Das Rohprodukt wurde in 20 ml Aethanol/Wasser (3:1) gelöst und mit 275 mg 10% Palladium auf Kohle 2 Stunden bei Raumtemperatur hydriert. Filtration über Filterhilfsmittel und Nachwaschen ergab reine 4-O-(β-D-Maltosyl)-α,α-D-trehalose (635 mg), $[\alpha]_D^{20}$ = +150,0° (c = 0,2, $H_2O$).

C. Eine Lösung von 1,0 g gut getrockneter 4-O-(β-D-Maltosyl)-α,α-D-trehalose in 25 ml absolutem Dimethylformamid wurden in Gegenwart von 5,845 g Schwefeltrioxid-Trimethylaminkomplex 18 Stunden bei 50°C gerührt, wobei ein zäher Sirup ausfiel. Aufarbeitung wie in Beispiel 1G. beschrieben ergab 2,63 g sulfatierte 4-O-(β-D-Maltosyl) -α,α-D-trehalose, S = 19,91%, DS etwa 2,8.

Beispiel 4

A. Zu einer Lösung von 4,0 g gut getrockneter 2,2',3,3',6'-Penta-O-benzyl-4,6-O-benzyliden-α,α-D-trehalose in 35 ml absolutem Dichlormethan und 3,1 ml Tetramethylharnstoff wurde in Gegenwart von 1,74 g Silbertrifluormethansulfonat bei -30°C eine Lösung von 6,67 g Deca-O-acetyl-α-D-maltotriosylbromid (Carbohydr. Res. 48, 197 (1976)) in 40 ml absolutem Dichlormethan zugetropft. Nach 3 Stunden bei Raumtemperatur wurde bei -30°C eine Zugabe von 3,4 g Deca-O-acetyl-α-D-maltotriosylbromid in 20 ml absolutem Dichlormethan sowie von 1 g 4Å-Molekularsieb vorgenommen. Nach 90 Stunden wurde aufgearbeitet. Chromatographie an Kieselgel mit Essigester/Hexan 3:2 als Elutionsmittel ergab 4,03 g (50%) 4'-(Deca-O-acetyl-β-D-maltotriosyl)-2,2',3,3',6'-penta-O-benzyl-4,6-O-benzyliden-α,α-D-trehalose als Sirup, $[\alpha]_D^{20}$ = +100,5° (c = 0,2, Dioxan).

B. Eine Lösung von 3,46g 4'-(Deca-O-acetyl-β-D-maltotriosyl)-2,2',3,3',6'-penta-O-benzyl-4,6-O-benzyliden-α,α-D-trehalose in 45 ml absolutem Methanol wurde in Gegenwart einer katalytischen Menge von wasserfreiem Natriumcarbonat 24 Stunden bei Raumtemperatur gerührt. Nach Filtration und Neutralisation mit saurem Ionenaustauscher wurde eingeengt und an Kieselgel mit Essigester/Methanol/Wasser 85:10:5 als Elutionsmittel chromatographiert. Die Produktfraktion (2,29 g, 86%) wurden in 240 ml Aethanol/Wasser (5:1) gelöst und in Gegenwart von 10% Palladium auf Kohle 3 Stunden bei Raumtemperatur hydriert. Filtration über Filterhilfsmittel und Nachwaschen ergab reine 4-O-(β-D-Maltotriosyl)-α,α-D-trehalose als Schaum (1,21 g, 100%), $[\alpha]_D^{20}$ = +172,5° (c=0,2, $H_2O$).

C. Eine Lösung von 500 mg gut getrockneter 4-O-(β-D-maltotriosyl)-α,α-D-trehalose in 10 ml absolutem Dimethylformamid wurden in Gegenwart von 2,85 g Schwefeltrioxid-Trimethylaminkomplex 18 Stunden bei 50°C gerührt, wobei ein zäher Sirup ausfiel. Aufarbeitung wie in Beispiel 1G. beschrieben ergab 1,32 g sulfatierte 4-O-(β-D-Maltotriosyl)-α,α-D-trehalose, S=19,5%, DS etwa 2,6.

Beispiel 5

A. Zu einer Lösung von 1 g gut getrockneter 2,2',3,3',6'-Penta-O-benzyl-4,6-O-benzyliden-α,α-D-trehalose in 1 ml absolutem Dichlormethan und 0,3 ml Tetramethylharnstoff wurde in Gegenwart von 442 mg Silbertrifluormethansulfonat bei -30°C eine Lösung von 1,19 g getrocknetem Hepta-O-acetyl-α-D-cellobiosylbromid (Ann. 435, 1 (1923)) in 5 ml absolutem Dichlormethan zugetropft. Nach 60 Stunden Rühren bei Raumtemperatur wurde über Filterhilfsmittel filtriert, mit Dichlormethan nachgewaschen, eingedampft und mit Dichlormethan/Aceton 24:1 als Elutionsmittel an Kieselgel chromatographiert. Es wurde 1,51 g (88%) reine 4'-O-(Hepta-O-acetyl-β-D-cellobiosyl)-2,2',3,3',6'-penta-O-benzyl-4,6,O-benzyliden-α,α-D-trehalose erhalten, $[\alpha]_D^{20}$ = +35,0° (c=0,3, $CHCl_3$).

B. Eine Lösung von 1,0 g 4'-O-(Hepta-O-acetyl-β-D-cellobiosyl)-2,2',3,3',6'-penta-O-benzyl-4,6-O-benzyliden-α-α-D-trehalose in 30 ml absolutem Methanol wurde in Gegenwart einer katalytischen Menge Natrium (einige mg) bei Raumtemperatur gerührt. Nach 2 Stunden wurde mit saurem Ionenaustauscher neutralisiert, eingedampft und in Chloroform/Methanol 9:1 über Kieselgel filtriert. Eindampfen ergab ein deacetyliertes Rohprodukt (764 mg), von dem 168 mg in 10 ml Aethanol/Wasser (4:1) in Gegenwart von 10% Palladium auf Kohle bei Raumtemperatur hydriert wurden. Nach 2,5 Stunden wurde über Filterhilfsmittel filtriert, nachgewaschen und eingedampft. Man erhielt 93 mg (100%) 4-O-(β-D-Cellobiosyl)-α,α-D-trehalose, $[\alpha]_D^{20}$ = +97° (c= 0,3, $H_2O$).

C. Eine Lösung von 72 mg 4-O-(β-D-Cellobiosyl)-α,α-D-trehalose in 5 ml wasserfreiem Dimethylformamid wurde in Gegenwart von Schwefeltrioxid-Trimethylaminkomplex 20 Stunden bei 50°C gerührt, wobei ein zäher Sirup ausfiel. Aufarbeitung wie in Beispiel 1G beschrieben ergab 176 mg sulfatierte 4-O-(β-D-Cellobiosyl)-α,α-D-trehalose, S=18,2%, DS etwa 2,7.

Beispiel 6

A. Zu einer Suspension von 1,0 g 2,2',3,3'-Tetra-O-benzyl-α,α-D-trehalose (Chem. Pharm. Bull. 30, 1169 (1982)) in 80 ml Toluol wurde 2,19 ml Bis-tributylzinnoxid gegeben und während 3,5 Stunden am Wasserabscheider unter Rückfluss erhitzt. Dabei wurde das Volumen um 40 ml reduziert. Dann wurden 157 mg Tetrabutylammoniumbromid und 0,84 ml Benzylbromid zugegeben und 30 Stunden bei 80°C gerührt. Nach der Zugabe der gleichen Menge Tetrabutylammoniumbromid und Benzylbromid wurde weitere 72 Stunden bei 80°C gerührt. Das Reaktionsgemisch wurde auf Eiswasser/Methylenchlorid gegossen und mit Natriumbicarbonatlösung und Wasser extrahiert. Nach Eindampfen der organischen Phasen wurden an Kieselgel mit Essigester/Hexan 1:2 als Elutionsmittel chromatographiert. Es wurde reine 2,2',3,3',6,6'-Hexa-O-benzyl-α,α-D-trehalose (1120 mg, 89%) als Sirup erhalten, $[\alpha]_D^{20}$ = +115,2° (c=0,5, Dioxan).

B. Zu einer Suspension von 1,0 g 2,2',3,3',6,6'-Hexa-O-benzyl-α,α-D-trehalose und 0,88 g Silbertriflat in 3 ml wasserfreiem Dichlormethan und 0,61 ml Tetramethylharnstoff wurde bei -30°C eine Lösung von 2,38 g Hepta-O-acetyl-a-D-maltosylbromid in 12 ml wasserfreiem Dichlormethan gegeben. Nach 10 Tagen bei Raumtemperatur wurde über Filterhilfsmittel filtriert, mit Dichlormethan gewaschen, eingedampft und das Rohprodukt an Kieselgel mit Aceton/Hexan 2:3 als Elutionsmittel chromatographiert. Es wurden 1,96 g reine 4,4'-Bis-O-(hepta-O-acetyl-β-D-maltosyl)-2,2',3,3',6,6'-hexa-O-benzyl-α,α-D-trehalose (82%) erhalten, Schmelzpunkt 85°C, $[\alpha]_D^{20}$ = +84,5° (c=0,2, Chloroform).

C. Eine Lösung von 1,0 g 4,4'-Bis-O-(hepta-O-acetyl-β-D-maltosyl)-2,2',3,3',6,6'-hexa-O-benzyl-α,α-D-trehalose in 40 ml Methanol/Dioxan (1:1) wurde bei Raumtemperatur eine katalytische Menge Natriummethylat gegeben. Nach 4 Stunden wurde mit saurem Ionenaustauscher neutralisiert, filtriert und eingedampft. Die deacetylierte Verbindung wurde in 50 ml Aethanol/Wasser (1:1) gelöst und in Gegenwart von 300 mg 10% Palladium auf Kohle bei Raumtemperatur hydriert. Nach 1 Stunde wurde über Filterhilfsmittel filtriert, mit Aethanol/Wasser (1:1) gewaschen und eingedampft. Es wurden 465 mg reine 4,4'-Bis-O-(β-D-maltosyl)-α,α-D-trehalose erhalten, $[\alpha]_D^{20}$ = +148° (c=0,3, $H_2O$).

D. Eine Lösung von 0,3 g 4,4'-Bis-O-(β-D-maltosyl)-α,α-D-trehalose in 15 ml wasserfreiem Dimethylformamid wurde in Gegenwart von 1,685 g Schwefeltrioxid-Trimethylaminkomplex 20 Stunden bei 50°C gerührt, wobei ein zäher Sirup ausfiel. Aufarbeitung wie in Beispiel 1G. beschrieben ergab 652 mg sulfatierte 4,4'-Bis-O-(β-D-maltosyl) -α,α-D-trehalose, S=18,93%, DS= 2,5.

Beispiel 7

A. Zu einer gekühlten Lösung (-80°C) von 618 mg 2,3,4,6-Tetra-O-acetyl-D-glucopyranosyltrichloracetimidat und 553 mg 2,2',3,3',6,6'-Hexa-O-benzyl-α,α-D-trehalose in 5 ml absolutem Dichlormethan wurde unter Argon 0,21 ml

Trifluormethansulfonsäureanhydrid gegeben. Nach 2,5 Stunden bei -10°C wurden bei -80°C nochmals 123 mg 2,3,4,6-Tetra-O-acetyl-D-glucopyranosyltrichloracetimidat und 41 ml Trifluormethansulfonsäureanhydrid zugegeben. Es wurde auf -10°C erwärmt, Triäthylamin zugegeben und das Lösungsmittel verdampft. Nach Codestillation mit Toluol wurde mit Toluol/Essigester 7:3 als Elutionsmittel an Kieselgel chromatographiert und 832 mg (86%) reine 4,4'-Bis-O-(2,3,4,6-tetra-O-acetyl-β-D-glucopyranosyl)-2,2',3,3',6,6'-hexa-O-benzyl-α,α-D-trehalose erhalten, $[\alpha]^{20}_{D}$ = +42° (c=0,3, Chloroform).

B. Eine Lösung von 696 mg 4,4'-Bis-O-(2,3,4,6-tetra-O-acetyl-β-D-glucopyranosyl)- 2,2',3,3',6,6'-hexa-O-benzyl-a,a-D-trehalose in 40 ml wasserfreiem Methanol wurde mit einer katalytischen Menge Natrium versetzt. Nach 20 Minuten bei Raumtemperatur wurde mit saurem Ionenaustauscher neutralisiert, filtriert und eingedampft. Filtration über Kieselgel mit Chloroform/Methanol 9:1 als Elutionsmittel ergab 480 mg deacetylierte Verbindung, die in 50 ml Aethanol/Wasser (4:1) in Gegenwart von 200 mg 10% Palladium auf Kohle bei Raumtemperatur hydriert wurde. Nach 2 Stunden wurde über Filterhilfsmittel filtriert, mit Aethanol/Wasser (1:1) gewaschen und eingedampft. Es wurden 250 mg 4,4'-Bis-O-(β-D-glucopyranosyl)-α,α-D-trehalose erhalten, $[\alpha]^{20}_{D}$ = +108° (c=0,2, $H_2O$).

C. Eine Lösung von 208 mg 4,4'-Bis-O-(β-D-glucopyranosyl)-α,α-D-trehalose in 5 ml absolutem Dimethylformamid wurde in Gegenwart von 1,22 g Schwefeltrioxid-Trimethylaminkomplex 20 Stunden bei 50°C gerührt, wobei ein zäher Sirup ausfiel. Aufarbeitung wie in Beispiel 1G. beschrieben ergab 520 mg sulfatierte 4,4'-Bis-O-(β-D-glucopyranosyl)-α,α-D-trehalose, S=19,52%, DS etwa 2,7.

Beispiel 8

A. Zu einer Lösung von 2,64 g am Hochvakuum getrockneter 2,2',3,3',6'-Penta-O-benzyl-4,6-O-benzyliden-α,α-D-trehalose und 1,29 g 2,3,4-Tri-O-acetyl-6-O-(2,3,4-tri-O-acetyl-6-desoxy-α-L-mannopyranosyl)-α-D-glucopyranosylbromid [Ber. 70, 1098-1101 (1938)] in 20 ml absolutem Dichlormethan wurden bei 0-5°C 0,52 g Silbertrifluormethansulfonat und sofort danach 0,6 ml absoluter Tetramethylharnstoff zugegeben. Nach 75 Minuten Weiterrühren bei 0-5°C wurde das Reaktionsgemisch über Dicalite abgenutscht, mit Dichlormethan nachgewaschen, das Filtrat zweimal mit gesättigter Natriumbicarbonatlösung gewaschen, über Magnesiumsulfat getrocknet und eingeengt. Der Rückstand wurde auf 85 g Kieselgel (70-230 mesh) mit Essigester/Hexan 1:4, 1:2 und 1:1 als Eluiermittel chromatographiert und ergab 839 mg (29%) O-(2,3,4-Tri-O-acetyl-6-desoxy-α-L-mannopyranosyl)-(1→6)-O-(2,3,4-tri-O-acetyl-β-D-glucopyranosyl)-(1→4)-2,2',3,3',6'-penta-O-benzyl-4,6-O-benzyliden-α,α-D-trehalose als Schaum, $[\alpha]^{20}_{D}$ = +32,2° (c = 0.5, $CHCl_3$).

B. Zu einer Lösung von 750 mg O-(2,3,4-Tri-O-acetyl-6-desoxy-α-L-mannopyranosyl)-(1→6)-O-(2,3,4-tri-O-acetyl-β-D-glucopyranosyl)-(1→4)-2,2',3,3',6'-penta-O-benzyl-4,6-O-benzyliden-α,α-D-trehalose in 1,5 ml Diäthyläther und 7,5 ml Methanol wurden bei Raumtemperatur 1,5 ml 1% Na in Methanol zugegeben und 3 Stunden weitergerührt. Die Reaktionslösung wurde dann mit saurem Ionenaustauscher (Amberlite IR-120) neutralisiert, 30 Minuten gerührt, abfiltriert, mit Methanol nachgewaschen und das Filtrat eingedampft. Der Rückstand wurde auf 27 g Kieselgel (70-230 mesh) mit Essigester/Methanol/Wasser 98:1:1 als Eluiermittel chromatographiert. Es wurden 380 mg (62%) deacyliertes Produkt erhalten, $[\alpha]^{20}_{D}$ = +7,0° (c = 0.1, $CHCl_3$). Davon wurden 360 mg (0,303 mmol) in 27 ml Aethanol/Wasser 2:1 in Gegenwart von 180 mg 10% Palladium auf Kohle bei Raumtemperatur hydriert. Nach 14 Stunden wurde das Reaktionsgemisch über Dicalite abgenutscht, mit Aethanol/Wasser nachgewaschen und das Filtrat eingeengt und am Hochvakuum getrocknet. Man erhielt 205 mg (100%) O-(6-Desoxy-α-L-mannopyranosyl)-(1→6)-O-β-glucopyranosyl-(1→4)-α,α-D-trehalose als amorphes Pulver, $[\alpha]^{20}_{D}$ = +9,3° (c = 0.1, $H_2O$).

C. Eine Lösung von 200 mg O-(6-Desoxy-α-L-mannopyranosyl)-(1→6)-O-β-glucopyranosyl-(1→4)-α,α-D-trehalose in 7 ml absolutem Dimethylformamid wurde mit 1,72 g Schwefeltrioxid-Trimethylaminkomplex versetzt und 20 Stunden bei 60-65°C unter Argon gerührt. Während dieser Zeit fiel ein harziger Niederschlag aus. Das Lösungsmittel wurde abdekantiert und der Rückstand in 11 ml 10%iger Natriumacetatlösung gelöst und am Wasserstrahlvakuum eingeengt. Der Rückstand wurde mehrmals (10x) in jeweils 50 ml Wasser aufgenommen und zur Entfernung von Trimethylamin eingedampft, dann zur Entfernung von Salzen gelchromatographiert (Sephadex® LH20, 150 g). Die Fraktionen mit dem sulfatierten Tetrasaccharid wurden eingedampft und lyophilisiert. Es wurden 320 mg (54%)

sulfatierte O-(6-Desoxy-α-L-mannopyranosyl)-(1→6)-O-β-D-glucopyranosyl-(1→4)-α,α-D-trehalose als amorphes Pulver erhalten. S = 19,62%, DS etwa 2,7.

Beispiel 9

A. Zu einer Lösung von 2,64 g am Hochvakuum getrockneter 2,2',3,3',6'-Penta-O-benzyl-4,6-O-benzyliden-α,α-D-trehalose -D-trehalose in 20 ml absolutem Dichlormethan wurden bei 0-5°C in rascher Reihenfolge 0,52 g Silbertrifluomethansulfonat, 1,40 g 2,3,4-Tri-O-acetyl-6-O-(2,3,6-tetra-O-acetyl-α-D-glucopyranosyl)-α-D-glucopyranosylbromid [Wolfram et al., J. Am. Chem. Soc., 71, 125-127 (1949)] und 0,6 ml absoluter Tetramethylharnstoff (5,0 mmol) zugegeben und 1 Stunde bei 0-5°C weitergerührt. Das Reaktionsgemisch wurde über Dicalite abgenutscht, mit Dichlormethan nachgewaschen, das Filtrat zweimal mit gesättigter Natriumbicarbonatlösung gewaschen, über Magnesiumsulfat getrocknet, abfiltriert und eingeengt. Der Rückstand wurde auf 85 g Kieselgel (70-230 mesh) mit Essigester/Hexan 1:4, 1:2 und 1:1 als Eluiermittel chromatographiert. Es wurden 740 mg (25%) O-(2,3,4,6-Treta-O-acetyl-α-D-glucopyranosyl)-(1→6)-O-(2,3,4-tri-O-acetyl-β-D-glucopyranosyl)-(1→4) -2,2',3,3',6'-penta-O-benzyl-4,6-O-benzyliden-α,α-D-trehalose als Schaum erhalten, $[\alpha]_D^{20}$ = +78,2° (c = 0.5, $CHCl_3$).

B. Zu einer Lösung von 660 mg O-(2,3,4,6-Tetra-O-acetyl-α-D-glucopyranosyl)-(1→6)-O-(2,3,4-tri-O-acetyl-β-D-glucopyranosyl)-(1→4)-2,2',3,3',6'-penta-O-benzyl-4,6-O-benzyliden-α,α-D-trehalose in 1,5 ml Diäthyläther und 7,5 ml Methanol wurden bei Raumtemperatur 1,32 ml 1% Na in Methanol zugegeben und 3 Stunden bei Raumtemperatur gerührt. Die Reaktionslösung wurde dann mit saurem Ionenaustauscher (Amberlite® IR-120) 10 Minuten gerührt, abfiltriert und mit Methanol nachgewaschen. Das Filtrat wurde eingedampft und auf 27 g Kieselgel (70-230 mesh) mit Essigester/Methanol/Wasser 96:2:2 als Eluiermittel chromatographiert. Es wurden 420 mg (79%) deacyliertes Produkt erhalten, $[\alpha]_D^{20}$ = +103,0° (c = 0.1, $CHCl_3$). Davon wurden 400 mg (0,33 mmol) in 30 ml Aethanol/Wasser 2:1 in Gegenwart von 200 mg 10% Palladium auf Kohle bei Raumtemperatur hydriert. Nach 14 Stunden wurde über Dicalite abgenutscht, mit Aethanol/Wasser nachgewaschen und das Filtrat eingeengt und am Hochvakuum getrocknet. Man erhielt 230 mg (100%) O-(α-D-Glucopyranosyl)-(1→6)-O-(β-D-glucopyranosyl)-(1→4) -α,α-D-trehalose als amorphes Pulver, $[\alpha]_D^{20}$ = +133,0° (c = 0.1, $H_2O$).

C. Eine Lösung von 213 mg O-(α-D-Glucopyranosyl)-(1→6)-O-(β-D-glucopyranosyl)-(1→4)-α,α-D-trehalose in 8 ml trockenem Dimethylformamid wurde mit 1,80 g Schwefeltrioxid-Trimethylaminkomplex 20 Stunden bei 60-65°C gerührt, wobei ein glasiger, harziger Niederschlag ausfiel. Nach Dekantieren des Lösungsmittels wurde der Rückstand in 11 ml 10%iger Natriumacetatlösung gelöst und am Wasserstrahlvakuum eingeengt. Der Rückstand wurde mehrmals (12x) in jeweils 50 ml Wasser gelöst und zur Entfernung von Trimethylamin jeweils eingedampft. Der Rückstand wurde zur Entfernung von Salzen gelchromatographiert (Sephadex® LH 20, 150 g) Produkt-Fraktionen wurden eingedampft und gefriergetrocknet. Es wurden 390 mg (~58%) sulfatierte O-α-D-Glucopyranosyl-(1→6) -O-β-D-glucopyranosyl-(1→4)-α,α-D-trehalose als amorphes Pulver erhalten, S = 20,36%, DS etwa 3,0.

Beispiel 10

A. Zu einer Lösung von 3,84 g 2,2',3,3',4,6,6'-Hepta-O-benzyl-α,α-D-trehalose [S. Kato, K. Yago, Bull. Chem. Soc. Jpn., 59, 411-414 (1986)] in 25 ml absolutem Dichlormethan wurden bei 0-5°C nacheinander und in rascher Folge 1,52 g Silbertrifluormethansulfonat, 4,13 g Hepta-O-acetyl-α-D-melibiosylbromid [Jeanes et al., J. Am. Chem. Soc., 75, 3667-3672 (1953)] in 15 ml absolutem Dichlormethan und 0,76 ml absoluter Tetramethylharnstoff zugegeben. Nach 2 Stunden Weiterrühren bei 0-5°C wurde das Reaktionsgemisch über Dicalite abgenutscht und mit Dichlormethan nachgewaschen. Das Filtrat wurde zweimal mit gesättigter Natriumbicarbonatlösung gewaschen, über Magnesiumsulfat getrocknet, abfiltriert, eingeengt und auf 350 g Kieselgel (70-230 mesh) mit Essigester/Hexan 1:4, 1:3 und 1:2 als Eluiermittel chromatographiert. Es wurden 2,93 g (47%) reine 4-O-(Hepta-O-acetyl-β-D-melibiosyl) -2,2',3,3',4,6,6'-hepta-O-benzyl-α,α-D-trehalose als Schaum erhalten, $[\alpha]_D^{20}$ = +93,4° (c = 0.5, $CHCl_3$).

B. Eine Lösung von 2,75 g 4-O-(Hepta-O-acetyl-β-D-melibiosyl)-2,2',3,3',4,6,6'-hepta-O-benzyl-α,α-D-trehalose in 5,5 ml Diäthyläther und 27 ml Methanol wurde bei Raumtemperatur mit 5,5 ml 1% Natrium in Methanol versetzt und 2,5 Stunden gerührt. Die Reaktionslösung wurde dann mit saurem Ionenaustauscher (Amberlite IR-120) 10

Minuten gerührt, abgenutscht und mit Methanol nachgewaschen. Das Filtrat wurde eingedampft und der Rückstand auf 75 g Kieselgel (70-230 mesh) mit Essigester/Methanol/Wasser 96:2:2 als Eluiermittel chromatographiert. Es wurden 1,53 g (68%) deacetyliertes Produkt erhalten, $[\alpha]_D^{20}$ = +115° (c = 0.2, $CHCl_3$). Davon wurden 1,296 g (1,0 mmol) in 40 ml Aethanol/$H_2O$ 3:1 in Gegenwart von 0,7 g 10% Palladium auf Kohle bei Raumtemperatur hydriert. Nach 8 Stunden wurde das Reaktionsgemisch über Decalite abgenutscht und mit Aethanol/Wasser nachgewaschen. Das Filtrat wurde eingeengt, am Hochvakuum getrocknet und ergab 0,69 g (100%) 4-O-(β-D-Melibiosyl) -α,α-D-trehalose als amorphes Pulver, $[\alpha]_D^{20}$ = +143,2° (c = 0.5, $H_2O$).

C. Eine Lösung von 660 mg 4-O-(β-D-Melibiosyl)-α,α-D-trehalose in 15 ml trockenem Dimethylformamid wurde mit 4,17 g Schwefeltrioxid-Trimethylaminkomplex versetzt und 20 Stunden bei 60-65°C gerührt, wobei nach ca. 3 Stunden ein zäher Sirup ausfiel. Das Lösungsmittel wurde abdekantiert und der Rückstand in 25 ml 10%iger Natriumacetatlösung gelöst und am Wasserstrahlvakuum eingeengt. Der Rückstand wurde mehrmals (12x) in jeweils 50 ml Wasser aufgenommen und zur Entfernung von Trimethylamin eingeengt. Der Rückstand wurde zur Entfernung von Salzen gelchromatographiert (Sephadex® LH 20, 150 g). Fraktionen mit sulfatiertem Tetrasaccharid wurden eingedampft und gefriergetrocknet. Es wurden 1,59 g (ca. 80%) sulfatierte 4-O-(β-D-Melibiosyl)-α,α-D-trehalose erhalten, $[\alpha]_D^{20}$ = +135,2° (c = 1.0, $H_2O$), S = 20,58%, DS etwa 3,0.

Beispiel 11

A. Zu einer Lösung von 1,74 g getrockneter 2,2',3,3',6'-Penta-O-benzyl-4,6-O-benzyliden-α,α-D-trehalose (Carbohydr. Res. 63, 51 (1978)) in 12,5 ml absolutem Dichlormethan wurde unter Argon und Lichtausschluss bei 0-5°C 0,37 g absoluter N,N,N,N-Tetramethylharnstoff und 0,76 g getrocknetes Silbertrifluormethansulfonat zugegeben. Eine Lösung von 1,92 g Acetobrom-D-lactose (J. Am. Chem. Soc. 37 1270 (1915)) in 7,5 ml Dichlormethan wurde zugetropft. Nach 1,5 Stunden Rühren bei 0-5°C wurde die Reaktionslösung über Kieselgur filtriert und mit Dichlormethan gewaschen. Die organischen Lösungen wurden mit Natriumbicarbonatlösung und Wasser gewaschen und über Magnesiumsulfat getrocknet. Chromatographie an Kieselgel mit Essigester/Hexan 1:2 als Elutionsmittel ergab 1,54 g reine 4'-O-(Hepta-O-acetyl-β-D-lactosyl)-2,2',3,3',6'-penta-O-benzyl-4,6-O-benzyliden-α,α-D-trehalose als weisses Harz, FAB-MS (1521,2 (M+Na)+).

B. Eine Lösung von 1,22 g 4'-O-(Hepta-O-acetyl-β-D-lactosyl)-2,2',3,3',6'-penta-O-benzyl-4,6-O-benzyliden-α,α-D-trehalose in 12,2 ml absolutem Methanol und 2,5 ml absolutem Aether wurde mit 3,1 ml 1%iger Natriummethylatlösung versetzt. Nach 5,5 Stunden bei Raumtemperatur wurde mit saurem Ionenaustauscher neutralisiert, filtriert und eingeengt. Chromatographie an Kieselgel mit Essigester/Methanol/Wasser 85:10:5 als Elutionsmittel ergab 0,91 g reine 4'-O-(β-D-Lactosyl)-2,2',3,3',6'-penta-O-benzyl-4,6-O-benzyliden-α,α-D-trehalose als weisses Harz, FAB-MS (1227,3 (M+Na)+).

C. Eine Lösung von 0,90 g 4'-O-(β-D-lactosyl)-2,2',3,3',6'-penta-O-benzyl-4,6-O-benzyliden-α,α-D-trehalose in 28 ml Aethanol/Wasser 3:1 wurde mit 0,5 g 10% Palladium auf Kohle 6 Stunden bei Raumtemperatur hydriert. Filtration über Filtrierhilfsmittel und Nachwaschen ergab nach Trocknung im Hochvakuum 0,51g reine 4'-O-(β-D-Lactosyl) -α,α-D-trehalose als weisses Harz, FAB-MS (689,0 (M+Na)+).

D. Eine Lösung von 0,49 g gut getrockneter 4'-O-(β-D-Lactosyl)-α,α-D-trehalose in 11,2 ml absolutem Dimethylformamid wurde in Gegenwart von 3,094 g Schwefeltrioxid-Trimethylaminkomplex 22 Stunden bei 65°C gerührt, wobei ein zäher Sirup ausfiel. Aufarbeitung wie im Beispiel 1G beschrieben ergab 1,1 g sulfatierte 4'-O-(β-D-Lactosyl)-α,-D-trehalose, S = 19,93%, DS etwa 2,9.

Beispiel 12

A. Zu einer Lösung von 1,43 g getrockneter 2,2',3,3',6'-Penta-O-benzyl-4,6-O-benzyliden-α,α-D-trehalose (Carbohydr. Res. 63, 51 (1978)) in 10,0 ml absolutem Dichlormethan wurde unter Argon und Lichtausschluss bei 0-5°C 0,30 g absoluter N,N,N,N-Tetramethylharnstoff und 0,625 g getrocknetes Silbertrifluormethansulfonat zugegeben. Eine Lösung von 1,70 g Acetobrom-D-gentiobiose (J. Am. Chem. Soc. 49, 3170 (1927)) in 6,0 ml Methylenchlorid wurde zugetropft. Nach 20 Stunden Rühren bei Raumtemperatur wurde die Reaktionslösung über Kieselgur filtriert und mit Dichlormethan gewaschen. Die organischen Lösungen wurden mit Natriumbicarbonatlösung und Wasser

gewaschen und über Magnesiumsulfat getrocknet. Chromatographie an Kieselgel mit Essigester/Toluol 1:2 als Elutionsmittel ergab 1,22 g reine 4'-O-(Hepta-O-acetyl-β-D-gentiobiosyl)-2,2',3,3',6'-penta-O-benzyl-4,6-O-benzyliden-α,α-D-trehalose als weisses Harz, FAB-MS (1521,2 (M+Na)+).

B. Eine Lösung von 1,22 g 4'-O-(Hepta-O-acetyl-β-D-gentiobiosyl)-2,2',3,3',6'-penta-O-benzyl-4,6-O-benzyliden-α,α-D-trehalose in 12,0 ml absolutem Methanol und 2,5 ml absolutem Aether wurde mit 2,44 ml 1%iger Natriummethylatlösung versetzt. Nach 5,5 Stunden bei Raumtemperatur wurde mit saurem Ionenaustauscher neutralisiert, filtriert und eingeengt. Chromatographie an Kieselgel mit Essigester/Methanol/Wasser 85:10:5 als Elutionsmittel ergab 0,50 g reine 4'-O-(β-D-Gentiobiosyl)-2,2',3,3',6'-penta-O-benzyl-4,6-O-benzyliden-α,α-D-trehalose als weisses Harz, FAB-MS (1227,4 (M+Na)+).

C. Eine Lösung von 0,48 g 4'-O-(β-D-gentiobiosyl)-2,2',3,3',6'-pentaO-benzyl-4,6-O-benzyliden-α,α-D-trehalose in 11 ml Aethanol und 3,7 ml Wasser wurde mit 0,27 g 10% Palladium auf Kohle 6 Stunden bei Raumtemperatur hydriert. Filtration über Filtrierhilfsmitel und Nachwaschen ergab nach Trocknung im Hochvakuum 0,26 g reine 4'-O-(β-D-Gentiobiosyl)-α,α-D-trehalose als weisses Harz, FAB-MS (689,0 (M+Na)+).

D. Eine Lösung von 0,20 g gut getrockneter 4'-O-(β-D-Gentiobiosyl)-α-α-D-trehalose in 5,0 ml absolutem Dimethylformamid wurde in Gegenwart von 1,26 g Schwefeltrioxid-Trimethylaminkomplex 20 Stunden bei 65°C gerührt, wobei ein zäher Sirup ausfiel. Aufarbeitung wie im Beispiel 1G beschrieben ergab 0,50 g sulfatierte 4'-O-(β-D-Gentiobiosyl)-α,α-D-trehalose, S = 20,25%, DS etwa 3,3.

Beispiel 13

A. Zu einer Suspension von 5 g 3-O-(β-D-Galactopyranosyl)-D-arabinose in 75 ml Pyridin wurden 50 ml Essigsäureanhydrid zugegeben und 6 Stunden bei Raumtemperatur gerührt. Die Reaktionslösung wurde am Wasserstrahlvakuum bei <40°C Badtemperatur eingeengt. Der sirupöse Rückstand wurde mit 200 ml Eiswasser versetzt und zweimal mit je 200 ml Essigester extrahiert. Die Extrakte wurden zweimal mit kalter 5%iger $H_2SO_4$,zweimal mit gesättigter Natriumbicarbonatlösung und einmal mit gesättigter Kochsalzlösung gewaschen, über Magnesiumsulfat getrocknet, abfiltriert und eingedampft und am Hochvakuum über Nacht getrocknet. Es wurden 8,84 g (91%) 1,2,4-Tri-O-acetyl-3-O-(2,3,4,6-tetra-O-acetyl-β-D-galactopyranosyl)-D-arabinose (nach NMR enthielt das Gemisch auch Furanose-Derivate) erhalten. 7,6 g davon wurden in 15 ml Dichlormethan gelöst, auf 0°C abgekühlt und mit 45 ml 33%ige Bromwasserstoffsäure in Essigsäure innert 15 Minuten versetzt und weitere 2 Stunden bei 0°C weitergerührt. Das Reaktionsgemisch wurde auf 250 ml Eiswasser gegossen und dreimal mit je 100 ml Dichlormethan extrahiert. Die Extrakte wurden zweimal mit je 100 ml Eiswasser, zweimal mit je 100 ml kalter gesättigter Natriumbicarbonatlösung gewaschen, über Magnesiumsulfat getrocknet, abfiltriert und bei <25°C eingedampft. Der Rückstand wurde auf 200 g Kieselgel (70-230 mesh) mit Dichlormethan/Diäthyläther 9:1 und 4:1 als Eluiermittel chromatographiert. Es wurden 4,15 g (53%) 2,4-Di-O-acetyl-3-O-(2,3,4,6-tetra-O-acetyl-β-D-galactopyranosyl) -β-D-arabinopyranosylbromid in Form eines Schaumes erhalten, $[\alpha]_D^{20}$ =-148,8° (c = 0.5, $CHCl_3$).

B. Zu einer Lösung von 3,52 g am Hochvakuum getrockneter 2,2',3,3',6'-Penta-O-benzyl-4,6-O-benzyliden-α,α-D-trehalose und 3,76 g 2,4-Di-O-acetyl-3-O-(2,3,4,6-tetra-O-acetyl-β-D-galactopyranosyl)-β-D-arabinopyranosyl)bromid in 30 ml absolutem Dichlormethan wurden bei 0-5°C nacheinander 2,0 ml absoluter Tetramethylharnstoff und 1,55 g Silbertrifluormethansulfonat zugegeben. Nach einer Stunde bei 0-5°C wurde das Reaktionsgemisch über Celite abgenutscht, mit Dichlormethan nachgewaschen, das Filtrat zweimal mit gesättigter Natriumbicarbonatlösung gewaschen, über Magnesiumsulfat getrocknet, abfiltriert und eingedampft. Der Rückstand wurde auf 190 g Kieselgel (70-230 mesh) mit Essigester/Hexan 1:2 und 1:1 als Eluiermittel chromatographiert. Es wurden 3,33 g (58%) O-(2,3,4,6-Tetra-O-acetyl-β-D-galactopyranosyl)-(1→3)-O-(2,4-di-O-acetyl-α-D-arabinopyranosyl)-(1→4)-2,2',3,3',6'-penta-O-benzyl-4,6-O-benzyliden-α,α-D-trehalose als Schaum erhalten. $[\alpha]_D^{20}$ = +42,0° (c = 0.5, $CHCl_3$).

C. Zu einer Lösung von 3,4 g O-(2,3,4,6-Tetra-O-acetyl-β-D-galacto-pyranosyl)-(1→3)-O-(2,4-di-O-acetyl-α-D-arabinopyranosyl)-(1→4)-2,2',3,3',6'-penta-O-benzyl-4,6-O-benzyliden-α,α-D-trehalose in 7 ml Diäthyläther und 35 ml Methanol wurden 3,4 ml 1% Na in Methanol zugegeben und 1,5 Stunden bei Raumtemperatur gerührt. Die Reaktionslösung wurde mit Ionenaustauscher (Amberlite® IR-120) neutralisiert, 15 Minuten gerührt, abfiltriert, mit Methanol nachgewaschen und das Filtrat eingedampft. Der Rückstand wurde auf 90 g Kieselgel (70-230 mesh) mit Essigester/Methanol/Wasser 93:5:2 als Eluiermittel chromatographiert. Es wurden 1,93 g (69%) deacetyliertes Produkt als Schaum erhalten. $[\alpha]_D^{20}$ = +52,0° (c = 0.5, $CHCl_3$). Davon wurden 1,80 g (1,53 mmol) in 60 ml Aethanol/Wasser 3:1 in Gegenwart von 1,0 g 10% Palladium auf Kohle bei Raumtemperatur hydriert. Nach 16 Stunden wurde das Reaktionsgemisch über Dicalite abgenutscht, mit Aethanol/Wasser nachgewaschen und das Filtrat eingedampft und getrocknet. Man erhielt 1,12 g eines Produktes, welches zur Nachreinigung mit Pyridin (22 ml) und Acetanhydrid (11 ml) acetyliert wurde. Nach 22 Stunden bei Raumtemperatur wurde das Reaktionsgemisch eingedampft und auf 85 g Kieselgel (70-230 mesh) mit Essigester/Hexan 1:1 und 2:1 als Eluiermittel chromatographiert. Es wurden 1,06 g (59%) O-(2,3,4,6-Tetra-O-acetyl-β-D-galactopyranosyl)-(1→3)-O-(2,4-di-O-acetyl-α-D-arabinopyranosyl)-(1→4) -hepta-O-acetyl-α,α-D-trehalose als Schaum erhalten. $[\alpha]_D^{20}$ = +68,0° (c = 0.4, $CHCl_3$). Davon wurden 1,02 g (0,86 mmol) in 30 ml Methanol/Dimethoxyäthan/Wasser 1:1:1 gelöst und tropfenweise mit 1%iger Natriummethylatlösung versetzt (pH 12-13) bis zur totalen Verseifung. Nach 6 Stunden wurde die Reaktionslösung mit Ionenaustauscher (Amberlite® IR-120) neutralisiert, 15 Minuten gerührt, abgenutscht, mit Methanol/Wasser nachgewaschen und das Filtrat eingedampft und am Hochvakuum getrocknet. Es wurden 480 mg (88%) O-(β-D-galactopyranosyl)-(1→3) -O-α-D-arabinopyranosyl-(1→4)-α,α-D-trehalose als amorphes Pulver erhalten. $[\alpha]_D^{20}$ = +9,4° (c = 0.1,$H_2O$).

D. Eine Lösung von 440 mg (0,69 mmol) O-β-D-galactopyranosyl-(1→3)-O-α-D-arabinopyranosyl-(1→4)-α,α-D-trehalose in 15 ml trockenem Dimethylformamid wurde mit 2,87 g Schwefeltrioxid-Trimethylaminkomplex versetzt und 20 Stunden bei 60-65°C unter Argon gerührt. Während dieser Zeit fiel ein harziger Niederschlag aus. Aufarbeitung wie im Beispiel 1G beschrieben ergab 960 mg (ca. 75%) sulfatierte O-β-galactopyranosyl-(1→3)-O-α-D-arabinopyranosyl-(1→4)-α,α-D-trehalose als amorphes Pulver, $[\alpha]_D^{20}$ = +58,8° (c = 0.5, $H_2O$). S = 19,24%, DS etwa 2,5.

Beispiel 14

Zur Herstellung einer Injektionslösung werden 5 mg einer Verbindung der Formel I und 9 mg Natriumchlorid in Wasser ad 1 ml gelöst. Die Lösung wird mit Ascorbinsäure (0,5 mg/ml) und Benzylalkohol (0,1 mg/ml) versetzt und dann sterilfiltriert.

**Patentansprüche**

1. Verbindungen der Formel

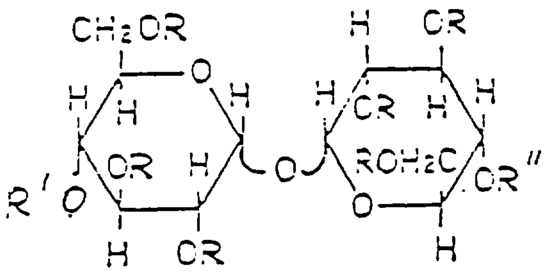

I

worin R Wasserstoff oder ein Rest $-SO_3M$; M ein physiologisch verträgliches Kation; R' ein equatorial oder quasiequatorial verknüpfter sulfatierter Mono-, Di- oder Trisaccharidrest oder ein axial verknüpfter sulfatierter Trisaccharidrest; und R" Wasserstoff oder ein equatorial oder quasiequatorial verknüpfter sulfatierter Mono-oder Disaccharidrest ist; wobei das Molekül höchstens 6 Monosaccharideinheiten enthält und im Mittel mindestens eine $-SO_3M$-Gruppe pro Monosaccharideinheit anwesend ist.

2. Verbindungen der Formel I gemäss Anspruch 1, worin R' ein β-glykosidisch verknüpfter sulfatierter Mono-, Di- oder Trisaccharidrest oder ein α-glykosidisch verknüpfter sulfatierter Trisaccharidrest; und R" Wasserstoff oder ein β-glykosidisch verknüpfter sulfatierter Mono-oder Disaccharidrest ist.

3. Verbindungen nach Anspruch 1, wobei R' ein sulfatierter β-Glucosyl-, β-Maltosyl-, β-Cellobiosyl- oder α- oder β-Maltotriosylrest ist.

4. Sulfatierte 4-O-(α-D-Maltotriosyl)-α,α-D-trehalose;

sulfatierte 4-O-(β-D-Glucopyranosyl)-α,α-D-trehalose;
sulfatierte 4-O-(β-D-Maltosyl)-α,α-D-trehalose;
sulfatierte 4-O-(β-D-Maltotriosyl)-α,α-D-trehalose;
sulfatierte 4-O-(β-D-Cellobiosyl)-α,α-D-trehalose;
sulfatierte 4,4'-Bis-O-(β-D-Maltosyl)-α,α-D-trehalose und
sulfatierte 4,4'-Bis-O-(β-D-Glucopyranosyl)-α,α-D-trehalose als Verbindungen nach Anspruch 1.

**5.** Sulfatierte O-(6-Desoxy-α-L-mannopyranosyl)-(1→6)-O-β-D-glucopyranosyl-(1→4)-α,α-D-trehalose;

sulfatierte O-α-D-glucopyranosyl-(1→6)-O-β-D-glucopyranosyl)-(1→4)-α,α-D-trehalose;
sulfatierte 4-O-(β-D-Melibiosyl)-α,α-D-trehalose;
sulfatierte 4'-O-(β-D-Lactosyl)-α,α-D-trehalose;
sulfatierte 4'-O-(β-D-Gentiobiosyl)-α,α-D-trehalose und
sulfatierte O-β-D-Galactopyranosyl-(1→3) -O-α-D-arabinopyranosyl-(1→4) -α,α-D-trehalose als Verbindungen nach Anspruch 1.

**6.** Verfahren zur Herstellung von Verbindungen gemäss Anspruch 1-5 dadurch gekennzeichnet, dass man ein entsprechendes Tri-, Tetra-, Penta-oder Hexasaccharid mit einem Sulfatierungsmittel bei 20-70°C behandelt und das Reaktionsprodukt als Salz isoliert.

**7.** Verwendung von Verbindungen gemäss Anspruch 1-5 als Wirkstoffe zur Herstellung pharmazeutischer Präparate zur Behandlung von arteriosklerotischen Erkrankungen.

**8.** Pharmazeutische Präparate, enthaltend eine Verbindung gemäss Anspruch 1-5 und übliche pharmazeutische Hilfsstoffe.

**9.** Verbindungen gemäss Anspruch 1-5 zur Anwendung als therapeutische Wirkstoffe.

## Claims

**1.** Compounds of the formula

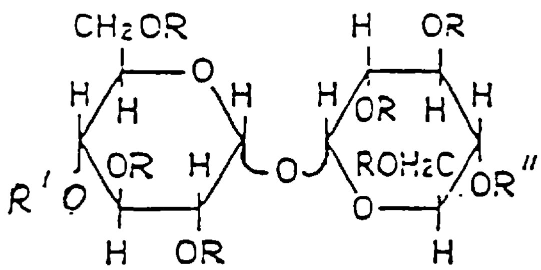

I

wherein R is hydrogen or a residue $-SO_3M$; M is a physiologically compatible cation; R' is a an equatorially or quasiequatorially linked sulphated mono-, di- or trisaccharide residue; or an axially linked sulphated trisaccharide residue; and R" is hydrogen or an equatorially or quasiequatorially linked sulphated mono- or disaccharide residue; whereby the molecule contains a maximum of 6 monosaccharide units and on average at least one $-SO_3M$ group is present per monosaccharide unit.

**2.** Compounds of formula I in accordance with claim 1, wherein R' is a β-glycosidically linked sulphated mono-, di- or trisaccharide residue or an α-glycosidically linked sulphated trisaccharide residue; and R" is hydrogen or a β-glycosidically linked sulphated mono- or disaccharide residue.

**3.** Compounds according to claim 1, wherein R' is a sulphated β-glucosyl, β-maltosyl, β-cellobiosyl or α- or β-maltotriosyl residue.

**4.** Sulphated 4-O-(α-D-maltotriosyl)-α,α-D-trehalose;
sulphated 4-O-(β-D-glucopyranosyl)-α,α-D-trehalose;
sulphated 4-O-(β-D-maltosyl)-α,α-D-trehalose;
sulphated 4-O-(β-D-maltotriosyl)-α,α-D-trehalose;

sulphated 4-O-(β-D-cellobiosyl)-α,α-D-trehalose;
sulphated 4,4'-bis-O-(β-D-maltosyl)-α,α-D-trehalose and
sulphated 4,4'-bis-O-(β-D-glucopyranosyl)-α,α,-D-trehalose as compounds according to claim 1.

**5.** Sulphated O-(6-desoxy-α-L-mannopyranosyl)-(1→6)-O-β-D-glucopyranosyl)-(1→4)-α,α-D-trehalose;
sulphated O-α-D-glucopyranosyl-(1→6)-O-β-D-glucopyranosyl-(1→4)-α,α-D-trehalose;
sulphated 4-O-(β-D-melibiosyl)-α,α-D-trehalose;
sulphated 4'-O-((β-D-lactosyl)-α,α-D-trehalose;
sulphated 4'-O-((β-D-gentiobiosyl)-α,α-D-trehalose and
sulphated O-β-D-galactopyranosyl-(1→3)-O-α-D-arabinopyranosyl-(1→4)-α,α-trehalose as compounds according to claim 1.

**6.** A process for the manufacture of compounds according to claim(s) 1-5 characterized by treating a corresponding tri-, tetra-, penta- or hexasaccharide with a sulphating agent at 20-70° C and isolating the reaction product as the salt.

**7.** The use of compounds according to claims 1-5 as active ingredients for the manufacture of pharmaceutical preparations for the treatment of arteriosclerotic disorders.

**8.** Pharmaceutical preparations, containing a compound according to claim(s) 1-5 and usual pharmaceutical adjuvants.

**9.** Compounds according to claim(s) 1-5 for use as therapeutically active substances.

**Revendications**

**1.** Composés de formule

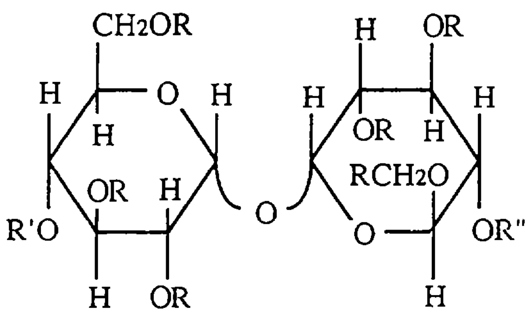

(I)

dans laquelle

- R représente l'hydrogène ou un groupe $-SO_3M$ ;
- M représente un cation acceptable pour les usages pharmaceutiques ;
- R' représente un radical de mono-, de di- ou de tri-saccharide sulfaté à liaison équatoriale ou quasi-équatoriale, ou un radical de trisaccharide sulfaté à liaison axiale ; et
- R" représente l'hydrogène ou un radical de mono- ou de di-saccharide sulfaté à liaison équatoriale ou quasi-équatoriale ;
  la molécule contenant au maximum six motifs de monosaccharides et en moyenne au moins un groupe $-SO_3M$ par motif de monosaccharide.

**2.** Composés de formule I de la revendication 1, dans laquelle

- R' représente un radical de mono-, di- ou tri-saccharide sulfaté à liaison β-glycosidique ou un radical de trisaccharide sulfaté à liaison α-glycosidique ; et
- R" représente l'hydrogène ou un radical de mono- ou di-saccharide sulfaté à liaison β-glycosidique.

**3.** Composés selon la revendication 1, pour lesquels

- R' représente un radical β-glucosyle, β-maltosyle, β-cellobiosyle ou α- ou β-maltotriosyle sulfaté.

**4.** Le 4-O-(α-D-maltrotriosyl)-α,α-D-tréhalose sulfaté ;
le 4-O-(β-D-glucopyrannosyl)-α,α-D-tréhalose sulfaté ;
le 4-O-(β-D-maltosyl)-α,α-D-tréhalose sulfaté;
le 4-O-(β-D-maltotriosyl)-α,α-D-tréhalose sulfaté ;
le 4-O-(β-D-cellobiosyl)-α,α-D-tréhalose sulfaté ;
le 4,4'-bis-O-(β-D-maltosyl)-α,α-D-tréhalose sulfaté ; et
le 4,4'-bis-O-(β-D-glucopyrannosyl)-α,α-D-tréhalose sulfaté, en tant que composés selon la revendication 1.

**5.** Le O-(6-désoxy-α-L-mannopyrannosyl)-(1→6)-O-β-D-glucopyrannosyl-(1→ 4)-α,α-D-tréhalose sulfaté ;
le O-α-D-glucopyrannosyl-(1→6)-O-β-D-glucopyrannosyl-(1→4)-α,α-D-tréhalose sulfaté;
le 4-O-(β-D-mélibiosyl)-α,α-D-tréhalose sulfaté ;
le 4'-O-(β-D-lactosyl)-α,α-D-tréhalose sulfaté ;
le 4'-O-(β-D-gentiobiosyl)-α,α-D-tréhalose sulfaté ; et
le O-β-D-galactopyrannosyl-(1→6)-O-α-D-arabinopyrannosyl-(1→4)-α,α-D-tréhalose sulfaté,
en tant que composés selon la revendication 1.

**6.** Procédé de préparation des composés selon les revendications 1 à 5, caractérisé en ce que l'on traite un tri-, tétra-, penta- ou hexa-saccharide correspondant par un agent sulfatant à des températures de 20 à 80°C et on isole le produit de réaction à l'état de sel.

**7.** Utilisation des composés selon les revendications 1 à 5 en tant que substances actives pour la préparation de compositions pharmaceutiques prévues pour le traitement des maladies artériosclérotiques.

**8.** Compositions pharmaceutiques contenant un composé selon les revendications 1 à 5 et des produits auxiliaires pharmaceutiques usuels.

**9.** Composés selon les revendications 1 à 5 pour l'utilisation en tant que substances thérapeutiquement actives.